# EUROPEAN PATENT APPLICATION

(11) **EP 1 787 575 A1**
(43) Date of publication of application: **23.05.2007**
(21) Application number: 05781393.3
(22) Date of filing: 02.09.2005
(51) Int. Cl.: A61B 1/00, G02B 23/24

(54) **ENDOSCOPE**

(30) Priority: 07.09.2004 JP 2004260131; 07.09.2004 JP 2004260133
(71) Applicant: Olympus Corporation, Tokyo 151-0072 (JP)
(72) Inventor: NOGUCHI, Toshiaki, 43-2, Hatagaya 2-chome, Shibuya-ku, Tokyo 151-0072 (JP); UCHIMURA, Sumihiro, 43-2, Hatagaya 2-chome, Shibuya-ku, Tokyo 151-0072 (JP); FURUKAWA, Tatsuya, 43-2, Hatagaya 2-chome, Shibuya-ku, Tokyo 151-0072 (JP); KAWAUCHI, Masahiro, 43-2, Hatagaya 2-chome, Shibuya-ku, Tokyo 151-0072 (JP); ONODA, Fumiyuki, 43-2, Hatagaya 2-chome, Shibuya-ku, Tokyo 151-0072 (JP); MORIYAMA, Hiroki, 43-2, Hatagaya 2-chome, Shibuya-ku, Tokyo 151-0072 (JP); TOYAMA, Ryuichi, 43-2, Hatagaya 2-chome, Shibuya-ku, Tokyo 151-0072 (JP); NIWA, Hiroshi, 43-2, Hatagaya 2-chome, Shibuya-ku, Tokyo 151-0072 (JP); KUROSHIMA, Hisashi, 43-2, Hatagaya 2-chome, Shibuya-ku, Tokyo 151-0072 (JP); HASEGAWA, Hitoshi, 43-2, Hatagaya 2-chome, Shibuya-ku, Tokyo 151-0072 (JP); SUZUKI, Eiri, 43-2, Hatagaya 2-chome, Shibuya-ku, Tokyo 151-0072 (JP); OGAWA, Akio, 43-2, Hatagaya 2-chome, Shibuya-ku, Tokyo 151-0072 (JP); GOCHO, Masanori, 43-2, Hatagaya 2-chome, Shibuya-ku, Tokyo 151-0072 (JP); ITO, Noriaki, 43-2, Hatagaya 2-chome, Shibuya-ku, Tokyo 151-0072 (JP); ITOYA, Satoshi, 43-2, Hatagaya 2-chome, Shibuya-ku, Tokyo 151-0072 (JP)
(74) Representative: von Hellfeld, Axel
(86) International application number: PCT/JP2005/016127
(87) International publication number: WO 2006/028019

(57) **Abstract**

An endoscope is provided which has good operability, such as ease of varying the rigidity of an insertion portion, as well as a lighter-weight bending mechanism for changing the shape of a bending portion. The endoscope according to the present invention comprises an insertion portion to be inserted into a subject, an electroconductive expanding/contracting member which can expand/contract the insertion portion in the lengthwise direction and which is for expanding/contracting according to the state of voltage application, and electrodes for applying the voltage supplied from a power source to the electroconductive expanding/contracting member.

## Description

### Technical Field

The present invention relates to an endoscope for inserting an insertion portion into a body cavity and performing endoscopy examination and so forth.

### Background Art

In recent years, endoscopes which can examine the inner portions of a subject such as a body cavity and so forth have become widely used in medical fields and so forth, by inserting an insertion portion.

An example of an endoscope which varies rigidity of the insertion portion so as to facilitate the insertion of the insertion portion into a body cavity and so forth is disclosed in Japanese Unexamined Patent Application Publication No. 10-10228 and Japanese Unexamined Patent Application Publication No. 10-276965.

With these Publications, configurations for variable rigidity are disclosed with a coil and a wire inserted into the coil, wherein the wire at the side of the hand is pulled, thus compressing the coil to make it rigid. In this case, in Japanese Unexamined Patent Application Publication No. 10-192223, an operating knob for pulling the wire is provided, and in Japanese Unexamined Patent Application Publication No. 10-276965, the configuration is such that the wire is pulled by turning an operating knob.

Thus with a conventional endoscope, the rigidity of the insertion portion is changed by gripping an operating knob by the hand and pulling or turning such knob.

Also, a mechanism is provided on the bending portion of the endoscope wherein the tip of the bending portion can be faced in a desired position by causing the bending portion to make a bending movement. An endoscope with such a mechanism is proposed in Japanese Unexamined Patent Application Publication No. 2003-38418, for example.

The present invention has taken the above-described problems into account, and it is an object thereof to provide an endoscope which enables easy modification of the rigidity of the insertion portion and smooth operability, as well as providing a more lightweight bending mechanism for modifying the shape of the bending portion.

### Disclosure of Invention

### Means for Solving the Problem

An endoscope according to the present invention includes an insertion portion for being inserted into a subject body cavity, an electroconductive expanding/contracting member which can expand and contract the insertion portion in the lengthwise direction, and which expands and contracts according to voltage application, and electrodes for applying voltage supplied from a power source to the electroconductive expanding/contracting member.

### Brief Description of the Drawings

Fig. 1 is an overall configuration diagram of an endoscope device having an endoscope according to a first embodiment of the present invention.
Fig. 2A is a length-wise cross-sectional view of an endoscope according to the first embodiment, shown in a state wherein driving voltage is not applied to a configuration with a variable rigidity mechanism.
Fig. 2B is a cross-sectional view along the 2B-2B line in Fig. 2A.
Fig. 3A is a length-wise cross-sectional view of an endoscope according to the first embodiment, shown in a state wherein driving voltage is applied to a configuration with a variable rigidity mechanism.
Fig. 3B is a cross-sectional view along the 3B-3B line in Fig. 3A.
Fig. 4A is length-wise cross-sectional view of an endoscope according to the first embodiment, shown in a state wherein driving voltage is applied to a configuration with a variable rigidity mechanism as a first modification example.
Fig. 4B is a cross-sectional view along the 4B-4B line in Fig. 4A.
Fig. 5A is length-wise cross-sectional view of an endoscope according to the first embodiment, shown in a state wherein driving voltage is not applied to a configuration with a variable rigidity mechanism as a first modification example.
Fig. 5B is a cross-sectional view along the 5B-5B line in Fig. 5A.
Fig. 6A is a cross-sectional view of the configuration of a portion of an insertion portion as a second modification example of the endoscope according to the first embodiment.
Fig. 6B is a cross-sectional view along the 6B-6B line in Fig. 6A.
Fig. 7 is a diagram illustrating the configuration of a variable rigidity mechanism provided on a flexible portion of an endoscope according to a second embodiment of the present invention.
Fig. 8A is a diagram illustrating the configuration of an insertion portion having a variable rigidity mechanism and control device in a state wherein driving voltage is not applied.
Fig. 8B is a diagram illustrating the configuration of a portion of the insertion portion and control device in a state wherein driving voltage is applied to an EPAM unit on the tip thereof.
Fig. 9 is a diagram illustrating a configuration of as a second modification example of an endoscope according to the second embodiment wherein a protrusion modifying mechanism is provided as well as the variable rigidity mechanism.
Fig. 10A is a diagram illustrating a schematic configuration of an insertion portion of an endoscope according to a third embodiment of the present invention.
Fig. 10B is a diagram illustrating the flexible portion shown in Fig. 10A in a state having multiple EPAM units protruded.
Fig. 11A is a diagram illustrating the configuration of a portion whereupon the EPAM units in Fig. 10 are provided.
Fig. 11B is a diagram illustrating an opening provided in a plate and the configuration of the EPAMs in Fig. 11A affixed so as to coat the opening from the inner side.
Fig. 12 is a diagram illustrating a schematic configuration of a sliding tube as a modification example of the endoscope according to the third embodiment.
Fig. 13 is a diagram illustrating the configuration of a twisting mechanism wherein the insertion portion is twisted by an operation on the side of the hand.
Fig. 14 is a cross-sectional diagram illustrating the configuration of the flexible portion in Fig. 13.
Fig. 15A is a perspective view of the configuration of the modification example in Fig. 13.
Fig. 15B is a side face view of the configuration of the modification example in Fig. 13.
Fig. 16 is a sideways cross-sectional view of the configuration of a cap.
Fig. 17 is an overall configuration of the endoscope according to a fourth embodiment of the present invention.
Fig. 18 is a cross-sectional view in the insertion axis direction of the endoscope in a bending portion of the endoscope relating to the fourth embodiment.
Fig. 19 is a cross-sectional view along the 19-19 line in Fig. 18.
Fig. 20 is a cross-sectional view in the insertion axis direction of the endoscope in the case of showing a cross-section of the external tube portion in Fig. 18.
Fig. 21 is a diagram showing the state of the endoscope according to the fourth embodiment being inserted into a body cavity.
Fig. 22 is a diagram showing the change in shape of a bending portion in the event of applying a generally similar voltage to all electrodes provided on the bending portion of the endoscope according to the fourth embodiment.
Fig. 23 is a cross-sectional view in the insertion axis direction of the endoscope of the case of having two bending portions which bend in four directions provided, in the bending portion of the endoscope according to the fourth embodiment.
Fig. 24 is a cross-sectional view in the insertion axis direction of the endoscope in the case of showing a cross-section of the external tube portion in Fig. 23.
Fig. 25 is a cross-sectional view in the insertion axis direction of the endoscope in the case of having a core line provided in the central portion within an inner tube, in the bending portion of the endoscope according to the fourth embodiment.
Fig. 26 is a cross-sectional diagram along the 26-26 line in Fig. 25, in the case of having a core line provided in the generally central portion within an inner tube, in the bending portion of the endoscope according to the fourth embodiment.
Fig. 27 is a cross-sectional view in the insertion axis direction of the endoscope, in the case of having a pipe provided in the generally central portion within an inner tube, in the bending portion of the endoscope according to the fourth embodiment.
Fig. 28 is a diagram illustrating a configuration in the case of applying the bending mechanism provided on the bending portion of the endoscope according to the fourth embodiment to a sliding tube.
Fig. 29 is a schematic diagram of the configuration of the endoscope main unit as a modification example of the endoscope according to the fourth embodiment.

### Best Mode for Carrying Out the Invention

Embodiments of the present invention will be described below with reference to the diagrams.

### (First Embodiment)

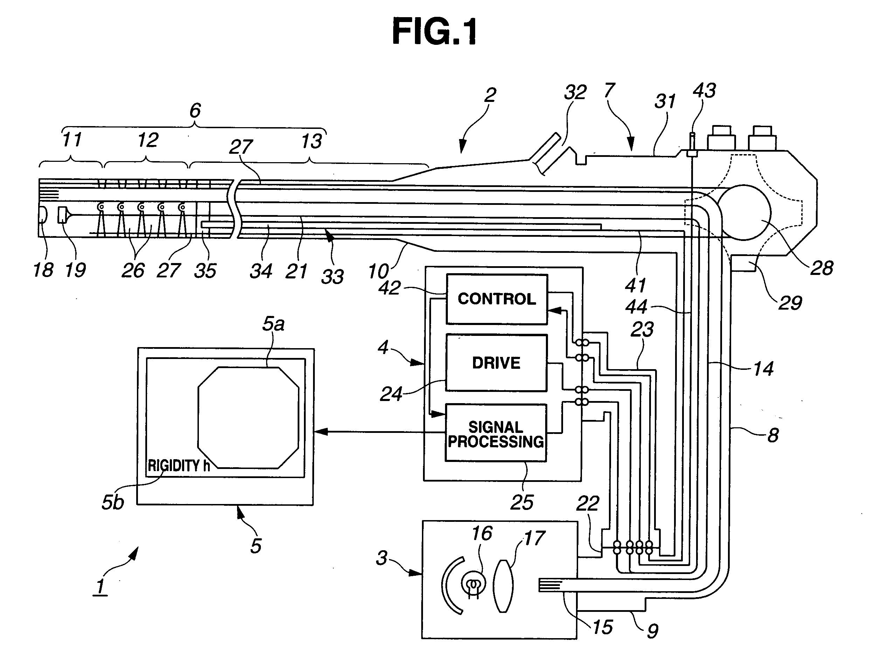

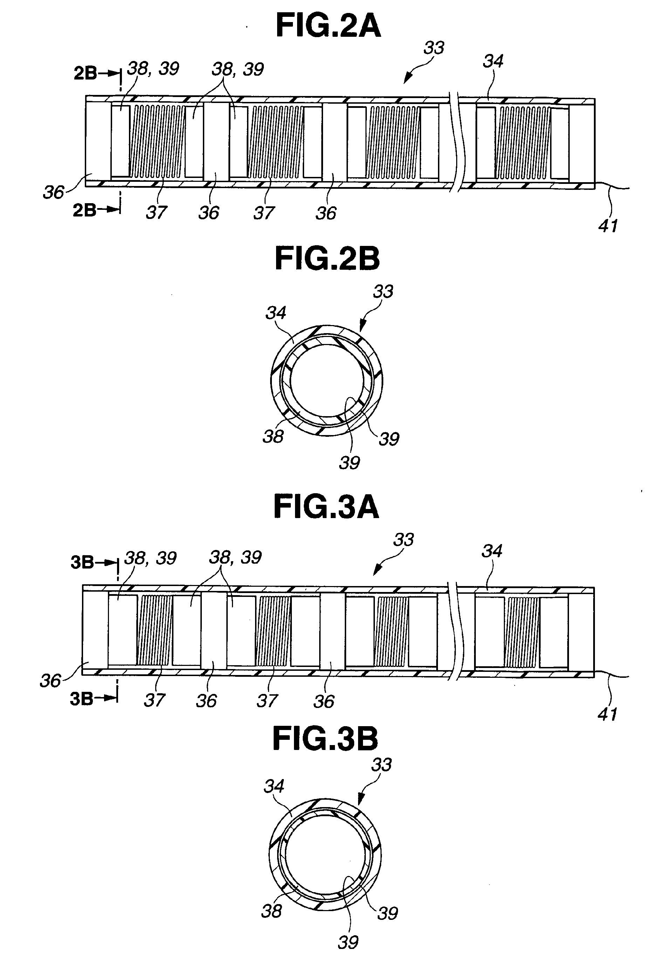

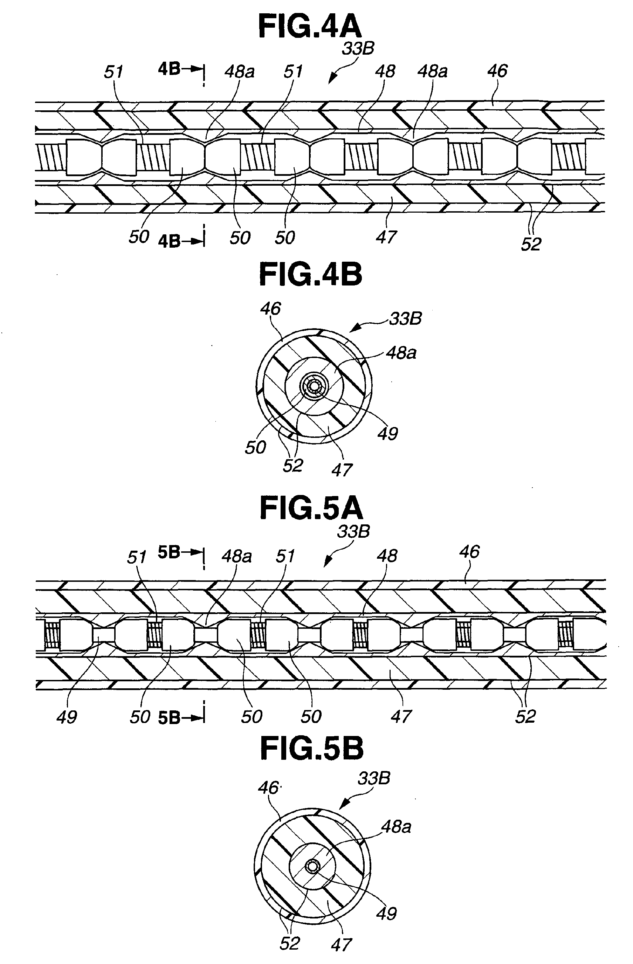

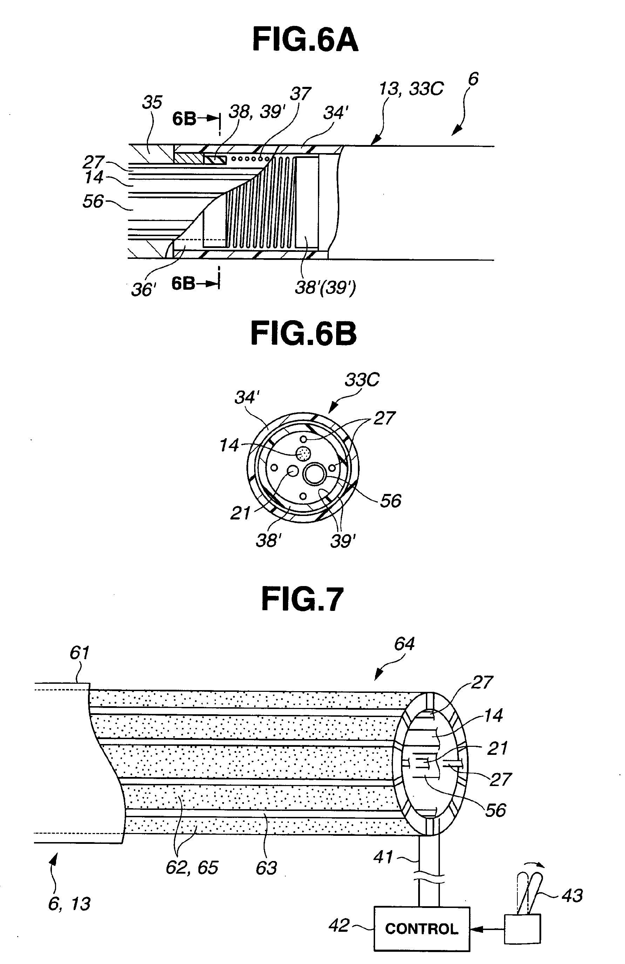

Fig. 1 through Fig. 6B relate to a first embodiment of the present invention; wherein Fig. 1 shows an overall configuration diagram of an endoscope device having an endoscope according to a first embodiment of the present invention. Fig. 2A, Fig. 2B, Fig. 3A, and Fig. 3B each show the configuration of a variable rigidity mechanism in a state of driving voltage being applied thereupon, and in a state of driving voltage not being applied, Fig. 4A, Fig. 4B, Fig. 5A, and Fig. 5B each show the configuration of a variable rigidity mechanism according to a first modification example in a state of driving voltage being applied thereupon, and in a state of driving voltage not being applied, and Fig. 6A and Fig. 6B show the configuration of a portion of an insertion portion as a second modification example.

Note that Fig. 2B is a cross-sectional view of the 2B-2B line in Fig. 2A, Fig. 3B is a cross-sectional view of the 3B-3B line in Fig. 3A, Fig. 4B is a cross-sectional view of the 4B-4B line in Fig. 4A, Fig. 5B is a cross-sectional view of the 5B-5B line in Fig. 5A, Fig. 6B is a cross-sectional view of the 6B-6B line in Fig. 6A.

As shown in Fig. 1, an endoscope device 1 comprises an electronic endoscope (written simply as endoscope) 2 according to a first embodiment having image-capturing means built-in, a light source device 3 for supplying illumination light to the endoscope 2, a signal processing device 4 for performing signal processing of an image-capturing signal output from the endoscope 2, and a color monitor 5 displaying a video signal output from the signal processing device 4 onto a screen.

The endoscope 2 comprises a slender insertion portion 6 to be inserted into a subject, a thick operating unit 7 which is consecutively provided on the rear end side of the insertion portion 6, and a universal cable 8 extending from the side portion of the operating unit 7, and a connector 9 is provided on the end portion of the universal cable 8, this connector 9 being detachably connected to the light source device 3.

The insertion portion 6 comprises a rigid tip portion 11 from the tip side, a bendable bending portion 12 on the rear end of this tip portion 11, and a flexible portion 13 which is long and flexible on the rear end of the bending portion 12, the rear end of this flexible portion 13 being linked to the front end of the operating unit 7. A fold-preventing member 10 having a fold-preventing function is provided on the rear side external periphery of the flexible portion 13 in a tapered shape.

A light guide 14, which has flexibility and which is made up of a fiber bundle having the function to transmit illumination light, is inserted into the insertion portion 6, operating unit 7, and universal cable 8, and by connecting a light guide connector portion 15 which is fixed so as to protrude from the connector 9 to the light source device 3, the illumination light from the lamp 16 within the light source device 3 is condensed with a lens 17 and supplied to the end face of the light guide connector portion 15.

The illumination light transmitted by this light guide 14 is emitted towards the front from the tip face which is fixed to an illumination window of the tip portion 11, and illuminates the subject such as a portion to be treated. The illuminated subject forms an optical image in an image-forming position by an objective lens 18 which is attached to the observation window provided adjacent to the illumination window on the tip portion 11. A charge-coupled device (abbreviated as CCD) 19 for example, serving as an image-capturing device having the function for photoelectric conversion is disposed at this image-forming position, and converts the image-formed optical image into an electric signal.

This CCD 19 is connected to one end of a signal cable 21, this signal cable 21 is inserted into the insertion portion 6 and so forth and the rear end thereof is connected to an electrical connector 22 of the connector 9, and is connected to the signal processing device 4 via an external cable 23 which is connected to an electrical connector 22.

The image-captured signal subjected to photoelectric conversion by the CCD 19 is read out when the CCD drive signal generated at a drive circuit 24 within the signal processing device 4 is applied to the CCD 19, and the image-captured signal is input into a signal processing circuit 25 within the signal processing device 4, and is converted into a standard video signal by the signal processing circuit 25. This standard video signal is input into the color monitor 5, and the endoscope image which is image-captured by the CCD 19 is displayed in color in an endoscope image display region 5a.

The bending portion 12 provided adjacent to the tip portion 11 is configured with a large number of ring-shaped bending pieces 26 which are rotatably linked with rivets and so forth in positions corresponding to a vertical and horizontal position to other adjacent bending pieces 26 while facing each other.

Also, the rear end of a bending wire 27 which is fixed to the tip-most bending piece 26 or the tip portion 11 is linked to a sprocket 28 within the operating unit 7, and a bending operating knob 29 for performing bending operations is attached to the shaft of this sprocket 28 (for simplification, Fig. 1 only shows the schematics of the bending mechanism in one directional pair of vertical or horizontal directions).

Then when the user performs an operation to turn the bending operating knob 29, one end of a pair of bending wires 27 positioned along the vertical direction or horizontal direction is pulled and the other end is relaxed, thus causing the bending portion 12 to bend toward the side of the pulled bending wire 27.

A gripping portion 31 is provided on the operating unit 7, farther toward the front side than the position at which the bending operating knob 29 is provided, and thus the surgeon can perform operations such as using the bending operating knob 29 with (a finger such as the thumb which is not used for gripping of) the one hand which is gripping the gripping portion 31.

Also, a treatment tool inserting opening 32 is provided farther toward the front side of the gripping unit 31, and by inserting a treatment tool through this treatment tool opening 32, the tip side of the treatment tool is protruded from a channel exit of the tip portion 11 via an internal treatment tool channel, and thus treatment such as the removal of a polyp can be performed.

Also, with the present embodiment, a variable rigidity mechanism 33 which can vary the rigidity of the flexible portion 13 for example is inserted within the insertion portion 6. This variable rigidity mechanism 33 is stored within a flexible tube 34, the tip of the flexible tube 34 is fixed to a rigid ring-shaped connecting tube 35 which connects, for example, the bending portion 12 and the flexible portion 13 within the insertion portion 6, the rear end thereof being fixed to a frame or the like on the inside of the gripping portion 31.

Note that the tip of the flexible tube 34 may also be fixed to the end-most bending piece 27 so as to duplicate the function of the connecting tube 35. The bending piece 26 which includes the connecting tube 35 is covered with an outer skin having elasticity, such as a rubber tube or the like.

Fig. 2A, Fig. 2B, Fig. 3A, and Fig. 3B each show the configuration of the variable rigidity mechanism 33 in a state of driving voltage being applied thereto, and in a state of driving voltage not being applied. Note that Fig. 2A shows a lengthwise cross-section of the variable rigidity mechanism 33 in a state of driving voltage not being applied thereto, and Fig. 2B shows a cross-section along the 2B-2B line in Fig. 2A. Also, Fig. 3A shows a lengthwise cross-section of the variable rigidity mechanism 33 in a state of driving voltage being applied thereto, and Fig. 3B shows a cross-section along the 3B-3B line in Fig. 3A.

Position fixing members 36 for fixing positions at predetermined intervals in the lengthwise direction are disposed within the flexible tube 34. Between adjacent position fixing members 36, a coil spring (spring coil) 37 serving as a variable rigidity member which varies so that the rigidity of bending is increased according to the compression state thereof by being compressed, and a ring-shaped electrically-conductive polymer artificial muscle (abbreviated as EPAM) 38 which is disposed on both sides in the lengthwise direction of the coil spring 37 and which is for acting (applying) compression force from both sides onto the coil spring 37, are disposed.

Each EPAM 38 serving as an electroconductive expanding/contracting member has electrodes 39 affixed to the inner circumferential surface and outer circumferential surface of the ring, and both electrodes 39 are electrically connected to a print pattern formed on the inner face of the tube 34 and on an outer circumferential position on the position fixing member 36.

Also, this print pattern is connected with the tip of a cable 41 at the rear end of the tube 34, and the rear end of this cable 41 is connected to a control circuit 42 within the signal processing device 4 via a cable within an external cable 23 connected to the electrical connector 22 of the connector 9.

Also, a rigidity varying stick 43 serving as instruction operating means for performing instruction operations to vary the rigidity is provided at a position near the gripping portion 31, for example, on the operating unit 7, and a cable 44 which is connected to this rigidity varying stick 43 is also connected to the control circuit 42.

Note that for example a potentiometer is provided on the base end of the rigidity varying stick 43, and the resistance values of the potentiometer change corresponding to a tilting operation of the rigidity varying stick 43. Then a signal corresponding to the tilt angle (corresponding to the change in resistance values) is input into the control circuit 42.

Then, a user such as a surgeon can tilt the rigidity varying stick 43 with the forefinger or the like of one hand which is gripping the gripping unit 31, thus a signal corresponding to the tilting angle is input into the control circuit 42, and an unshown CPU within the control circuit 42 generates driving voltage corresponding to the tilting angle.

Specifically, the greater the tilting angle, the greater drive voltage is generated. This drive voltage is applied to the electrodes 39 on the EPAM 38 configuring the variable rigidity mechanism 33 via the cable 41.

With a situation in which driving voltage is not applied as shown in Fig. 2A, the coil spring 37 is in a soft state not subjected to compression force, and is in a low rigidity state as to external bending force. Conversely, with the situation in Fig. 3A wherein driving voltage is applied, the coil spring 37 disposed between the EPAMs 38 are compressed by being pressed from both sides and become in a tightly wound state by the EPAM 38 expanding in the lengthwise direction.

In a state thus compressed by being pressured from both directions, changing shape is difficult, and so the rigidity as to external bending force of the variable rigidity mechanism 33 is increased. Thus, rigidity as to external bending force of the flexible portion 13 in which the variable rigidity mechanism 33 is inserted can be increased.

Note that the CPU in the control circuit 42 transmits information corresponding to the rigidity which the variable rigidity mechanism 33 sets with the driving voltage to the signal processing circuit 25. The signal processing circuit 25 superimposes the information corresponding to rigidity with the video signal corresponding to the image which is image-captured by the CCPD19, and outputs this.

Then, the information corresponding to rigidity is displayed in a rigidity information display portion 5b which is near the endoscope image display region 5a on the color monitor 5. With this display, the user can easily understand the currently set rigidity state by observing the endoscope image.

Note that Fig. 1 shows that the currently set rigidity value is h in the rigidity information display portion 5b.

Thus according to the present embodiment, the user can perform instruction operations for varying the rigidity by tilting the rigidity varying stick 43 with an forefinger or the like of the hand gripping the gripping portion 31, and can easily change the rigidity of the flexible portion 13 with such instruction operations.

Accordingly, for example, in the case of inserting the insertion portion 6 of the endoscope 2 from the anus to deep side within the colon, the rigidity can be changed and so insertion work can be performed smoothly. In such a case with a conventional example, performing an operation to change the rigidity could not be done with the gripping hand, and has had to be done with the other hand, thus lowering operability.

Also, with a conventional example, in the case of increasing rigidity, force for pulling a rigidity varying wire has been necessary, and there was the problem of needing a greater operating force.

Conversely, with the present embodiment, operations can be performed with a forefinger or the like of the gripping hand, thus enhancing operability.

With the present embodiment, electrical compressing driving means (compression applying means) are provided wherein an electrical signal is applied to the EPAM 38, the EPAM 38 is extended, and with such extension, compression force is applied to the coil spring 37 serving as a variable rigidity member and increasing the rigidity thereof. Therefore, work to change rigidity can be performed easily without requiring a great operating force, by simply performing tilting operations of the rigidity varying stick 43 serving as the instruction operating means.

Thus, according to the present embodiment, operability for insertion work and so forth can be greatly improved.

Note that the present embodiment has a configuration where EPAMs 38 are disposed on both sides of each coil spring 37, but a configuration may be used wherein one of the EPAMs is not used, and one end of the coil spring 37 is adjacent to a position fixing member 36. Also, a further simple configuration wherein the position fixing member 36 is omitted may also be used.

Also, the present embodiment uses a coil spring 37 as a rigidity varying member for changing the rigidity of the bending by compression force being applied, but not being limited to this, for example, an elastic member in a pipe shape for example may be used.

Fig. 4A, Fig. 4B, Fig. 5A, and Fig. 5B show a variable rigidity mechanism 33B as a first modification example of the present embodiment.

With this variable rigidity mechanism 33B, a tube-shaped EPAM tube 47 disposed so as to be fitted within a cylindrically shaped sheath (tube) 46 which does not expand/contract easily and which is flexible, and further within this EPAM tube 47, a generally cylindrically shaped pressing member 48 is enclosed.

On the inner side of the pressing member 48, a sliding piece 50 and a coil spring (spring coil) 51 are disposed on the outer side of the innermost guide tube 49.

The end portion outer circumference on one side of the generally ring-shaped sliding pieces 50 has a cutout portion formed therein wherein a cone shape or a rounded cone shape is cut out. Then the sliding pieces 50 are disposed so that the end faces of the cutout portions are facing each other, and a wedge-shaped protruding portion 48a of the pressing member 48 is disposed in the space formed by both cutout portions formed by the sliding pieces 50 which are adjacent to one another in this case.

Also, a coil spring 51 is disposed between the sliding pieces 50 of which the each of the side faces not providing a cutout portion are facing one another.

The pressing member 48 is in a general tube shaped which can change shape in the radius direction. Protruding portions 48a which protrude toward the inner side of the radius are provided at predetermined spacing at positions on the inner circumferential surface, and each of the protruding portions 48a is disposed so as to be close to, or to lightly make contact with, the cutout portions of the sliding pieces 50.

Also, a coil spring 51 which changes so as to become more rigid by a compressed state is disposed between adjacent sliding pieces 50 on the side without cutout portions provided, and both ends of the coil spring 51 press the sliding pieces 50 with a weak force. In this state, the coil spring 51 is in an easily bendable state with low rigidity.

Also, electrodes 52 are provided on the inner circumferential surface and outer circumferential surface of the EPAM tube 47, and both electrodes 52 are connected to the control circuit 42 via the cable 41 shown in Fig. 1 from the rear end of the EPAM tube 47.

Fig. 4A and Fig. 4B show a cross-sectional configuration of the variable rigidity mechanism 33B in the state of driving voltage being applied to the electrodes 52 of the EPAM tube 47 from the control circuit 42, wherein the cross-sectional configuration with the driving voltage turned OFF or the driving voltage at a smaller value changes from Fig. 4A and Fig. 4B to Fig. 5A and Fig. 5B.

In other words, normally, driving voltage is applied to the electrodes 52 on the outer circumferential surface and inner circumferential surface of the cylindrical EPAM tube 47, and the EPAM tube 47 expands to the state where the thickness thereof becomes thinner.

If the driving voltage is lowered from this state, EPAM tube 47 attempts to return to the original thickness. At this time, expansion of the outer circumferential surface side is restricted by the sheath 46, and so the EPAM tube 47 becomes thicker on the inner circumferential surface side, the pressing member 48 changes shape to have a contracted diameter, and in this event the sliding pieces 50 compress in the direction of pressing (compressing) the coil springs 51 at the locations of the protruding portions 48a.

The coil spring 51 portions increase rigidity by contracting, and also the adjacent sliding pieces 50 become difficult to bend and increase rigidity from the pressure from the pressing member 48.

With the above-described first embodiment or first modification example of the first embodiment, a variable rigidity mechanism 33 or 33B with a small-sized crosswise cross-section is provided within the insertion portion 6, but a variable rigidity mechanism 33C may also be formed on the outer skin portion of the insertion portion 6 as in a second modification example, as will be described below.

Fig. 6A and Fig. 6B show a variable rigidity mechanism 33C which is formed by applying a larger size crosswise cross-section of the variable rigidity mechanism 33 of the first embodiment to the outer skin portion of the flexible portion 13.

The tip of a tube 34' enclosing the variable rigidity mechanism 33C is fixed to the rear end of the connecting tube 35, for example, as shown in Fig. 6A. This tube 34' forms the outer skin of the flexible portion 13.

Position fixing members 36' are disposed at predetermined spacing on the inner side of the tube 34' in the lengthwise direction thereof, and in between the adjacent position fixing members 36', ring-shaped EPAMs 38' are disposed so as to sandwich the coil spring 37' from both sides.

Each EPAM 38' has electrodes 39' affixed to the inner circumferential surface and outer circumferential surface of the ring, and both electrodes 39' are electrically connected to a print pattern formed on the inner face of the tube 34' and on an outer circumferential position of the position fixing member 36'.

Also, this print pattern is connected with the tip of the cable 41 shown in Fig. 1 at the rear end of the tube 34', and the rear end of this cable 41 is electrically connected to a control circuit 42 within the signal processing device 4.

As shown in Fig. 6B serving as the 6B-6B line cross-sectional view of Fig. 6A, a bending wire 27 for bending the bending portion 12 is inserted in the vertical or horizontal direction for example, on the inner side of the variable rigidity mechanism 33C. Also, items built in to the insertion portion, such as a light guide 14, signal cable 21, channel tube 56 and so forth are inserted into the inner side of the variable rigidity mechanism 33C.

With such a configuration as well, the user can perform instruction operations for varying the rigidity by tilting the rigidity varying stick 43 with an forefinger or the like of the hand gripping the gripping portion 31, and by such instruction operations, can easily change the rigidity of the flexible portion 13.

Note that Fig. 6A and Fig. 6B are shown with an example applied to the variable rigidity mechanism 33 of Fig. 2A and Fig. 2B, but it is clear that a similar application could also be made to the variable rigidity mechanism 33B of Fig. 4A and Fig. 4B, by increasing the inner diameter of the guide tube.

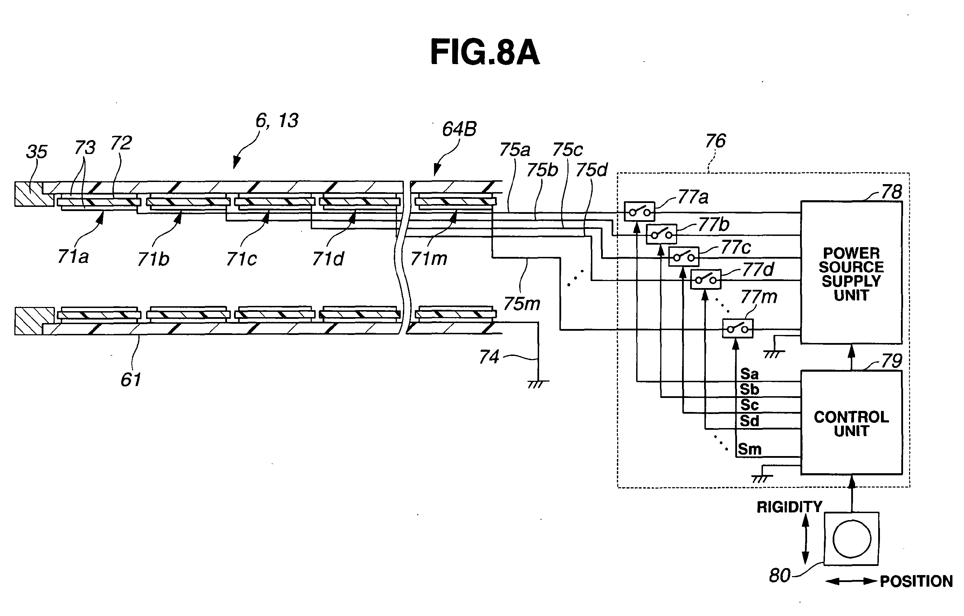

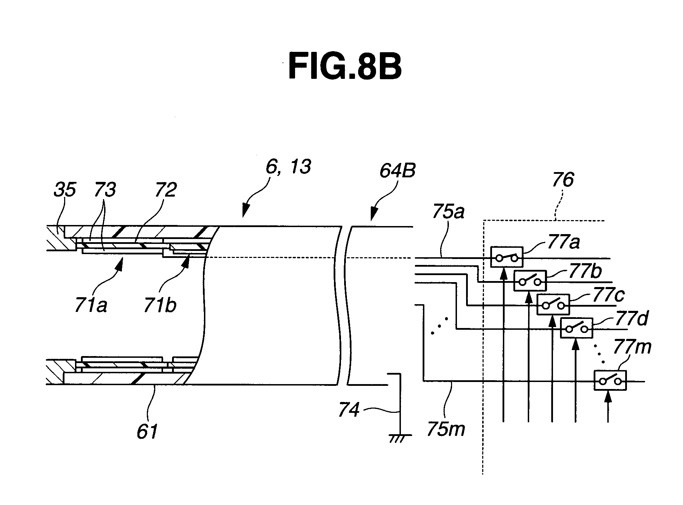

Note that in the above-described embodiment and modification example, with the variable rigidity mechanisms 33 through 33C within the flexible portion 13, the rigidity in the lengthwise direction is varied in the same way throughout the length thereof. However, in an embodiment to be described later (specifically, the variable rigidity mechanism 64B shown in Fig. 8A or Fig. 8B and the control device 76 thereof), the rigidity near an arbitrary position in the lengthwise direction can be varied.

For example in the case of the configuration in Fig. 2A and 2B, the electrodes 39 on the inner circumferential surface of the EPAM 37 disposed on both sides of each coil spring 37 are each connected to the control circuit 42 via separate cables, and further, selection means for selecting the EPAM 37 in positions on which driving voltage is to be applied can be provided as instruction operating means, and the selection signal of the selection means can be input into the control circuit 42.

### (Second Embodiment)

Next, a second embodiment according to the present invention will be described with reference to Fig. 7. Fig. 7 shows the configuration of the insertion portion of an endoscope according to the second embodiment of the present invention.

With the present embodiment, the flexible portion 13 of the insertion portion 6 is covered with an external skin tube 61, and a cylindrical shaped variable rigidity mechanism 64, which is made up of an EPAM 62 serving as an electroconductive expanding/contracting member and an insertion filling member 63, is formed on the inner side of the external skin tube 61.

This variable rigidity mechanism 64 is formed by linking multiple band-shaped EPAMs 62 and insertion filling members 63 which are disposed in the small spatial portions between the adjacent EPAMs 62 and which are capable of expanding/contracting, linking these in a perimeter direction such that a tube shape is formed. Note that the insertion filling members 63 can be made up of an elastic adhesive or the like.

Electrodes 65 are provided on the inner circumferential surface and outer circumferential surface of each EPAM 62 which are disposed parallel in the lengthwise direction on the insertion portion 6 in a band shape, and the various electrodes 65 are each standardized for those on the inner circumferential surface side and the outer circumferential surface side at the rear end of each EPAM 62, for example, and are connected to the control circuit 42 via a cable 41, as with the first embodiment.

Also, the control circuit 42 is connected to a rigidity varying stick 43 provided on the operating unit, and the control circuit 42 applies driving voltage for varying rigidity to the EPAM 62 via the cable 41 according to the tilt angle, upon the user performing an operation to tilt the rigidity varying stick 43.

The EPAMs 62 are extended in the circumferential direction (becomes thinner in the thickness direction) by driving voltage being applied thereto, and this presses the insertion filling members 63, pressing the adjacent EPAM 62 such that the rigidity thereof is increased.

With the first embodiment, the variable rigidity mechanism 33 is made up of an EPAM 38 serving as compression applying means for expanding from an electrical signal being applied thereto and generating compression force, and a coil spring 37 or the like serving as a rigidity varying member wherein the rigidity thereof varies due to the compression force being applied thereto. However, with the present embodiment, the EPAM 62 holds both function (that is to say, the function of compression applying means and a rigidity varying member).

Note that on the inner side of the cylindrically shaped variable rigidity mechanism 64, similar to the case of Fig. 6A and Fig. 6B, items built in to the insertion portion, such as a light guide 14, signal cable 21, channel tube 56 and so forth, are inserted. According to the present embodiment with such a configuration, similar to the case of Fig. 6A and Fig. 6B in the first embodiment, the user can perform instruction operations for varying the rigidity by tilting the rigidity varying stick 43 with a forefinger or the like of the hand gripping the gripping portion 31, and the control circuit 42 applies, according to the tilting angle, the driving voltage for varying rigidity to the EPAM 62 via the cable 41.

Then, as described above, the adjacent EPAMs 62 are pressured, and changed so that the rigidity thereof is increased, facilitating varying the rigidity of the flexible portion 13.

The present embodiment has the same advantages as those of the first embodiment, and also has the advantage of having the same functions as the first embodiment with a simpler configuration.

Note that in Fig. 7, the EPAM 62 is divided into multiple parts in the circumferential direction and the multiple EPAMs 62 are disposed in the lengthwise direction of the insertion portion 6, and insertion filling members 63 made up of an adhesive or the like are disposed between the adjacent EPAMs 62. However, instead of providing the insertion filling members 63, for example an EPAM without providing an electrode 65 may be provided. In this case, costs can be lowered since formed with a cylindrical shaped EPAM.

Also, instead of disposing the multiple EPAMs 62 in the lengthwise direction of the insertion portion 6 in parallel, the EPAMs 62 can be disposed in a spiral shape as to the inner circumferential surface of the outer skin tube 61, thus forming a variable rigidity mechanism with a one-line or two-line EPAM.

In this case, electrodes can be limited to one location (of a facing portion) or two locations. Thus, varying rigidity can be performed along the entire length of the portion having formed a variable rigidity mechanism such as the flexible portion 13 and so forth.

Next, the configuration of a first modification example of the present embodiment will be described, with reference to Fig. 8A and Fig. 8B. Fig. 8A illustrates the configurations of the insertion portion in which a variable rigidity mechanism is provided, and the control device in a state wherein driving voltage is not applied. Fig. 8B illustrates the configuration of a portion of the insertion portion and control device in a state wherein driving voltage is applied to an EPAM unit on the tip thereof, for example.

As shown in Fig. 8A and Fig. 8B, the tip of the outer skin tube 61 of the flexible portion 13 of the insertion portion 6 is fixed to the connecting tube 35. A variable rigidity mechanism 64B is formed on the inner side of the outer skin tube 61 by multiple EPAM units 71a, 71b, 71c, 71d, ... 71m with a predetermined length in the lengthwise direction as an increment, linked with an unshown elastic adhesive and so forth.

In this case, each EPAM unit 71i (i = a through m) is linked so as to be adjacent in the lengthwise direction at a slight distance from one another. In other words, normally, each EPAM unit 71i is in a state wherein pressuring force is not acting thereupon, and in this state, the flexible portion 13 is in a state of easily varying shape and has a low rigidity.

Each EPAM unit 71i is made up of a ring-shaped EPAM 72 and an electrode 73 provided on the inner circumferential surface and outer circumferential surface thereof, and the electrode 73 on the outer circumferential surface is connected with the entire outer circumferential surface with an unshown leading line or the like for the electrode 73 to become conductive.

Then the EPAM unit 71i is connected to a ground potential of a power source supplying unit 78 by a common signal line 74 which is connected to the electrode 73 on the outer circumferential surface of the end-most EPAM unit 71m.

On the other hand, the respective electrodes 73 provided on the inner circumferential surface at each EPAM unit 71i are each connected to the tip of the signal lines 75i, and the rear end of the signal lines 75i are connected to the power source supplying unit 78 via switches 77i within the control device 76 provided external to the endoscope.

Also, each switch 77i is turned ON/OFF by an ON/OFF control signal Si from the control unit 79.

Also, the control unit 79 can be configured such that an arbitrary switch 77i is turned ON/OFF according to an instruction operation with the instruction operating means by a track ball 80, for example, provided in a position easily operable on the operating unit 7.

By rotating the track ball 80 in a horizontal direction, for example, the user can select switches 77a through 77m to turn from OFF to ON according to the rotation angle thereof. Then by the user performing an operation to push in the track ball 80, the control unit 79 outputs a corresponding control signal Si to turn the selected switch 77i from OFF to ON.

As will be described below, each of the EPAM units 71i pressures the end face of an adjacent EPAM unit 71k (k = i-1, i+1) by expanding in the lengthwise direction of the insertion portion 6 by driving voltage being applied thereto, thus increasing the rigidity thereof.

Accordingly, by the user rotating the track ball 80 in a horizontal direction and selecting the switch 77a through 77m to turn ON, the rigidity at an arbitrary position in the EPAM units 71a through 71m which are disposed in the lengthwise direction of the flexible portion 13 can be changed (the "position" shown in Fig. 8A along the arrow in the horizontal direction near the track ball 80 shows that the position at which to change rigidity can be selected and set).

Also, by rotating the track ball 80 in a vertical direction, the control unit 79 performs control to vary the power source voltage which is output from the power source supplying unit 78. For example, in the case that the track ball 80 is rotated in an upward direction, the control unit controls the driving voltage output from the power source supplying unit 78 to be increased.

Conversely, if the track ball 80 is rotated in a downward direction, the control unit controls the driving voltage output from the power source supplying unit 78 to be decreased. Also, by increasing the driving voltage to be applied, the value of rigidity to be set can be increased.

Fig. 8A shows a state of all of the switches 77a through 77m being OFF, and in the case that the track ball 80 is operated and for example only switch 77a is turned ON, the driving voltage is applied to the EPAM unit 71 a via the switch 77a which is turned ON, as shown in Fig. 8B.

The ring-shaped thickness of the EPAM unit 71a becomes thinner and expands in the lengthwise direction. Also, the tip of the EPAM unit 71 a becomes in a state of pressuring the connecting tube 35, and the rear end of the EPAM unit 71a becomes in a state of pressuring the tip of the adjacent EPAM unit 71b, thus increasing the rigidity of the EPAM unit 7a portion. In actuality, a portion of the adjacent EPAM unit 71b also has a change in the rigidity thereof.

In this case, by rotating the track ball 80 in an upper direction, the value of the driving voltage can be increased, the proportion of the EPAM unit 71a to be expanded can be increased, and so the rigidity thereof can be increased further.

According to the first modification example of the present embodiment which has such a configuration and action, the rigidity of a portion at an arbitrary position in the lengthwise direction of the flexible portion 13 can be easily changed. Accordingly, insertion operability is improved.

Note that with the first modification example, the coil spring 37 described in the first embodiment can be disposed between the adjacent EPAM units 71i, 71i+1 (i+1 indicates b in the case that i = a), and thus the rigidity can be changed by the compression force acting primarily on a coil spring 37.

In this case as well, the rigidity of an arbitrary coil spring 37 portion in the lengthwise direction of the insertion portion 6 can be changed.

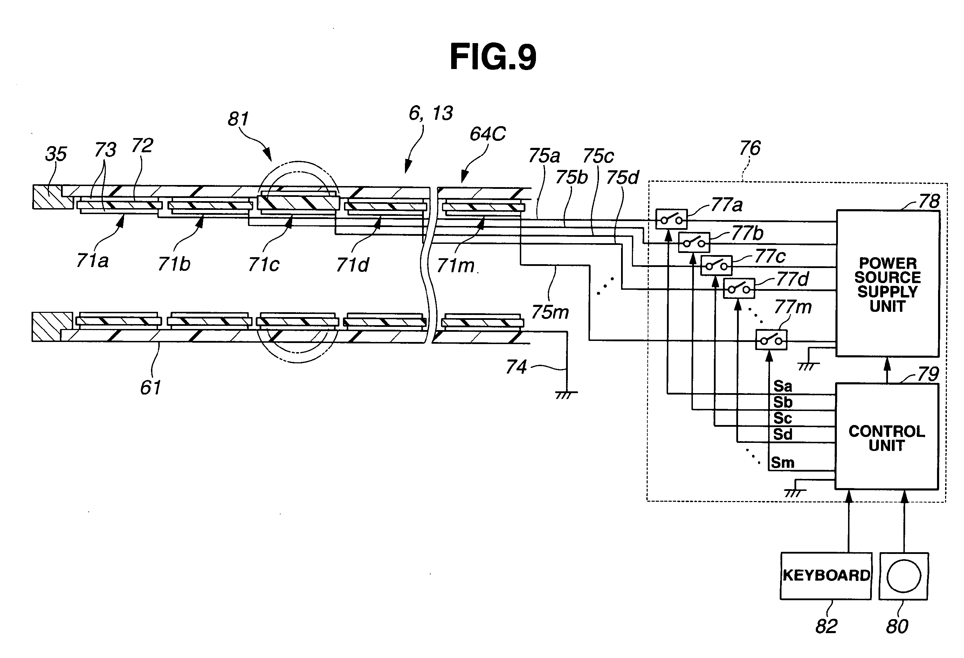

A second modification example of the present embodiment will be described with reference to Fig. 9. The variable rigidity mechanism 64C formed on the flexible portion 13 of the insertion portion 6 of this endoscope is configured with a protrusion varying mechanism 81 for changing the shape of the EPAM units 71c and so forth disposed in the lengthwise direction at predetermined spacing for example, so as to protrude outside in the radius direction, in the variable rigidity mechanism 64B which is provided on the flexible portion 13 shown in Fig. 8A and Fig. 8B.

Specifically, the EPAM units 71c and so forth (represented with 71j) disposed at predetermined spacing (intervals) in the lengthwise direction of the flexible portion 13 have a thickness of the EPAM 72 which is greater than others, so when driving voltage is applied, the shape change can be greater.

Also, the thickness of the EPAM unit 71j is made to be thick on the outer circumferential surface side, and so protrude in a step fashion on the outer circumferential surface side more than the adjacent EPAM units 71i (specifically, 71b, 71d and so forth, omitting 71c and so forth). Accordingly, the thickness of the outer skin tube 61 at the outer side becomes thinner by an amount equivalent to this thickness, and thus an outer skin is formed with a configuration facilitating shape changing.

As described above, the thickness of the outer skin tube 61 of the portion protruding toward the outer circumferential surface side (in other words the EMPAM unit 71j) becomes thinner in a stepped manner, so the thin portion of this outer skin tube 61 is more flexible than the thicker portions, and thus has a configuration facilitating shape changing.

Also, if the driving voltage is applied to the EPAM unit 71i by operating the track ball 80 on the hand side, the EPAM units 71a, 71b, 71d and so forth with the same configuration as the first modification example of the present embodiment will change rigidity as described with the first modification example of the present embodiment.

Conversely, if driving voltage is applied to the EPAM unit 71j such as the EPAM unit 71c and so forth of a thicker portion by the operation of the track ball 80, the thinner outer skin tube 61 can be deformed toward the outside in the radius direction, and so this portion can be deformed so as to cause the protruding portion protruding from the outer circumferential surface as shown by the dashed-two dotted line so as to be capable of protruding and retracting. Note that by stopping the application of driving voltage, the protruding portion disappears.

Thus with the present modification example, besides the action of varying rigidity by the variable rigidity mechanism 64B according to the first modification example of the present embodiment, the outer circumferential surface can be made to protrude and retract, and so insertion work and so forth can be performed more smoothly.

In other words, with a lower digestive system endoscope, in the case of being inserted into the colon, there are cases wherein insertion work can be performed more smoothly if the endoscope is fixed within the colon, however with conventional examples, fixing has been difficult, and a balloon sheath or the like has been necessary to use for affixing.

Conversely, with the present modification example, a portion of the EPAM unit 71c and so forth which has been instructed to operate can be protruded (expanded) from the outer circumferential surface of the tube-shaped insertion portion 6, by instruction operations by the track ball 80 on the hand side, and so the insertion portion 6 can be easily fixed to the colon wall and so forth. Accordingly, insertion work and so forth can be performed more smoothly.

Also, with the present modification example, a keyboard 82 or the like, for inputting instructions to instruct time intervals for applying the driving voltage and application time for applying the driving voltage to the EPAM units 71c and so forth provided at predetermined spacing in the lengthwise direction of the insertion portion 6, is connected to the control unit 79.

When the user performs instruction input to the keyboard 82 for the time intervals to temporally protrude the EPAM units 71c and so forth, the control unit 79 uses time measuring means such as an internal timer or the like, and applies the driving voltage at the instructed time intervals to the thick EPAM units 71c and so forth, and also stops applying the driving voltage after the instructing application time has passed. Then the thick EPAM units 71c and so forth protrude in the radius direction toward the outside direction at the instructed time intervals, and after the instructed application time has passed, the protrusions disappear.

Thus, for example by repeating the protrusion and protrusion release of the thick EPAM unit 71c and so forth, synchronizing with the peristalsis of the body for example, the insertion of the insertion portion 6 can be performed more smoothly.

Also, rather than repeating application and application stopping of the driving voltage simultaneously to the entire thick EPAM unit 71c and so forth, application and application stopping of the driving voltage can be performed in the order of disposal in the lengthwise direction of the insertion portion 6, shifting by the instructed time.

In this case also, the insertion work can be performed more smoothly.

In the second modification example of the present embodiment, the EPAM units 71c and so forth are provided in a ring shape, but the ring can be formed to be divided in the circumferential direction, a selection made from the multiple divided parts in the circumferential direction, and driving voltage can be selectively applied to the selected parts. Thus, portions protruding in the circumferential direction can also be controlled. The third embodiment described below has a similar configuration to this.

### (Third Embodiment)

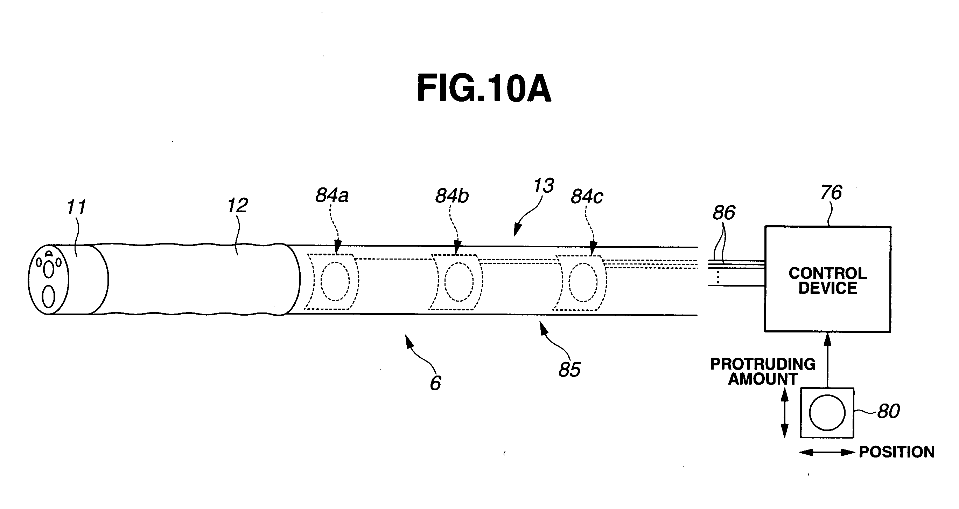

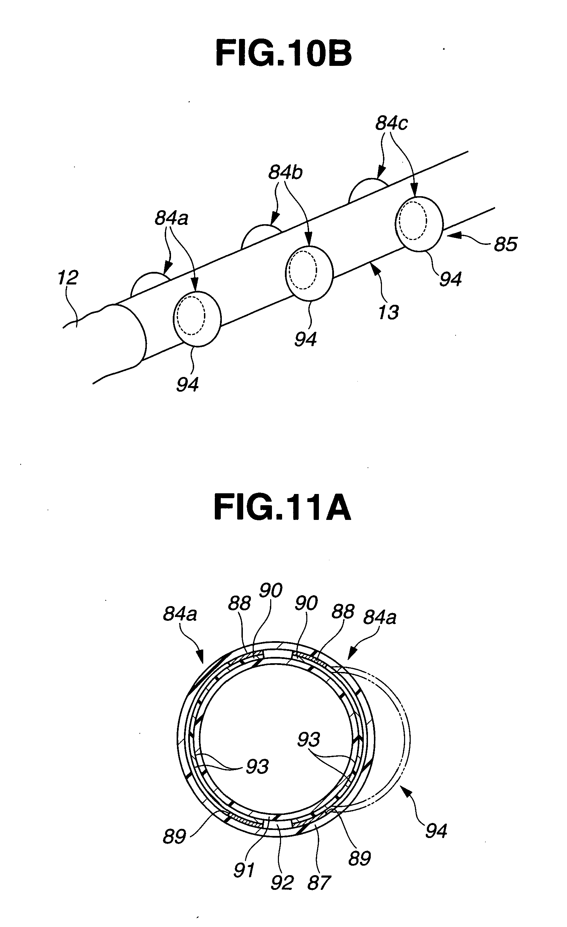

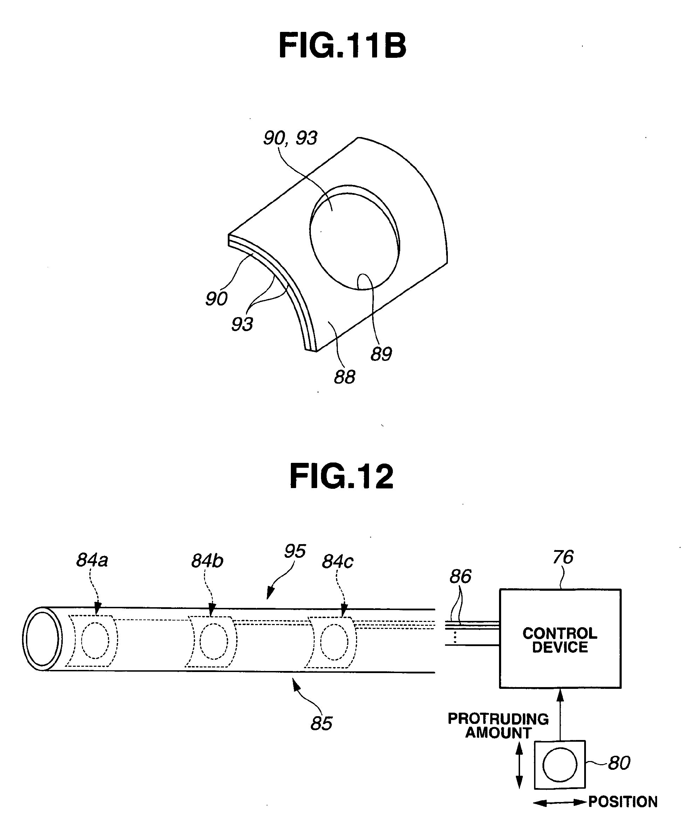

Next, the third embodiment of the present invention will be described with reference to Fig. 10A, Fig. 10B, Fig. 11A, and Fig. 11B. Fig. 10A and Fig. 10B show the configuration of a protrusion varying mechanism provided on the insertion portion of the endoscope according to the third embodiment of the present invention. The present embodiment has the following configuration with the object of improving operability of insertion work.

With the present embodiment as shown in Fig. 10A, EPAM units 84a, 84b, 84c, ... are provided at predetermined spacing along the lengthwise direction on the flexible portion 13 and form a protrusion varying mechanism 85. These EPAM units 84a, 84b, 84c, ... are connected to the control device 76 with a configuration such as that shown in Fig. 8 for example, via a cable 86.

Then, by operating the track ball 80 connected to the control device 76, driving voltage can be applied to an arbitrary EPAM unit 84k (k = a, b, c...), and so a protrusion portion (or a protruding portion) 94 can be formed by causing the EPAM unit to expand, such as shown in Fig. 10B.

Note that Fig. 10B shows a state of the flexible portion 13 shown in Fig. 10A wherein multiple EPAM units 84a, 84b, 84c are caused to protrude.

Fig. 11A shows a crosswise cross-sectional view a the EPAM unit 84a portion, and Fig. 11B shows an opening 89 provided on a plate 88 wherefrom a tube 87 forming the outer skin is removed, and an EPAM 90 which is attached so as to cover this opening 89 from the inner side.

As shown in Fig. 11A, a plate 88 in a general half-circle cylinder shape, providing the openings 89 on both sides in the left and right direction for example, and an EPAM 90 in a general half-circle cylinder shape so as to cover the openings 89, are disposed between the tube 87 forming the outer skin and the inner tube 91 disposed on the inner side, and are fixed with a linking member 92 at the upper edge and lower edge, for example.

Also, electrodes 93 are each provided on the inner circumferential surface and outer circumferential surface of each EPAM 90, and each are connected to the cable 86.

The solid line in Fig. 11A is in a state of not applying the driving voltage to the electrode 93 of the EPAM 90, and by applying the driving voltage, the EPAM 90 is made to be thinner in the thickness direction, and also by causing this to expand in the direction orthogonal to the thickness direction, the expanded EPAM 90 can be deformed so as to protrude outside the opening 89 from the opening 89, as shown with the dashed-two dotted line.

Note that in the case of applying driving voltage and causing the EPAM 90 to expand, the outer skin tube 87 on the outer circumferential surface thereof also is pressured and deformed by the EPAM 90 into the shape protruding from the outer circumferential surface.

According to the present embodiment with such a configuration, a protrusion varying mechanism 85 is provided which enables the EPAM units 84k to protrude so as to be capable of protruding/retracting at arbitrary positions along the lengthwise direction of the insertion portion 6, and therefore as described with the second modification example according to the second embodiment, the insertion portion 6 can be easily fixed to the colon wall and so forth, and thus operability of insertion work can be improved.

Fig. 12 shows a modification example, and shows the protrusion varying mechanism 85 which is applied to the flexible portion 13 in Fig. 10A as applied to the sliding tube 95. According to this modification example, a currently known endoscope insertion portion can be inserted through this sliding tube 95, thus enabling fixing to a colon wall and so forth, improving operability of insertion work.

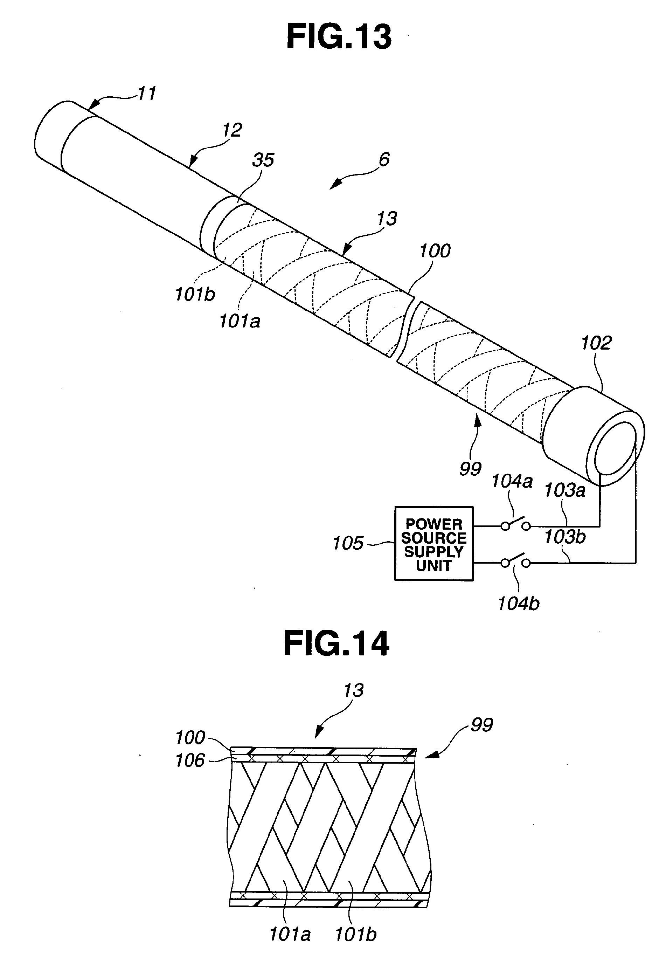

Incidentally, a configuration such as that shown in Fig. 13 may be used, wherein a twisting mechanism 99 is provided with the objective of easily performing a twisting operation as to the insertion portion 6. With the insertion portion 6 of the endoscope shown in Fig. 13, EPAMs 101a and 101b serving as electroconductive expanding/contracting members, which make up a band-shaped pair, each wrap around the inner side of the outer skin 100 (and braid 106) of the flexible portion 13 in a spiral manner to form the twisting mechanism 99.

The directions for wrapping the EPAMs 101 a and 101 b in a spiral manner are each wrapped in the opposite directions of a left direction and a right direction.

These doubled EPAMs 101 are connected to cables 103a, 103b at a cap 102 to which the rear end is fixed, and these cables 113a and 103b are each connected to the power source supplying unit 105 via switches 104a and 104b.

Fig. 14 shows a cross-sectional configuration of the flexible portion 13 in Fig. 12.

The EPAMs 101a and 101b in a braided band shape are built in, in double, to the inner side of the outer skin 100, and each EPAM is wrapped in the opposite directions of the left direction and the right direction.

Then, by operating the switches 104a and 104b from OFF to ON, the flexible portion 13 can be twisted in the OFF EPAM 101 side by expanding the EPAM 101 where the driving voltage has turned ON.

Thus, by having a configuration wherein a twisting mechanism 99 for twisting the insertion portion 6, the operability during endoscopy examination can be improved. For example, in a colon examination, the surgeon can use techniques to smoothly perform insertion in the event of inserting the endoscope into the deep portions of the colon. Of these techniques, the action of twisting the insertion portion of the endoscope is the most frequently performed action in endoscopy examination, and also this action places a greater burden on the surgeon.

In the configuration shown in Fig. 13, the insertion portion 6 can be twisted easily by operating the ON/OFF switches 104a and 104b on the hand side, and therefore the burden on the surgeon can be lessened at time of twisting. In other words, there is the advantage of improved operability for endoscopy examination.

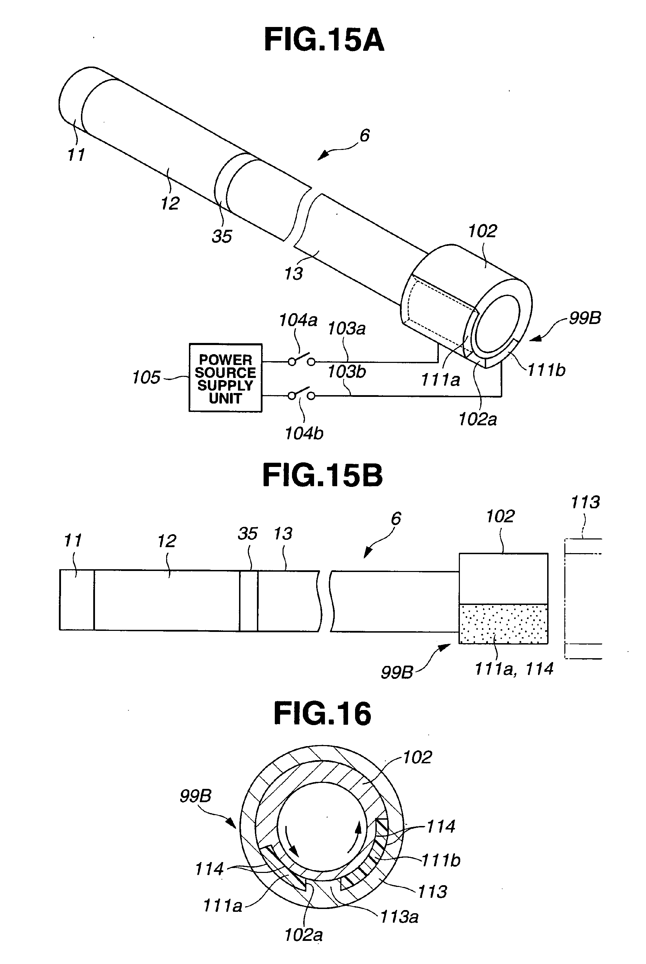

Fig. 15A and Fig. 15B show the insertion portion 6 provided with a modified example of a twisting mechanism 99B. Note that Fig. 15A shows a perspective view of the insertion portion 6, and Fig. 15B shows a side face view of the insertion portion 6.

In the case of Fig. 13, the EPAMs 101 a and 101b are provided spirally in opposite directions to one another within in flexible portion 13, but with the modified examples shown in Fig. 15A and Fig. 15B, the external surface of the cap 102 provided on the rear end of the flexible portion 13 is cut away by approximately half, for example, and the EPAMs 111a and 111b are attached facing on the left and right sides at such cutaway portions.

Note that the cutaway portion 102a between the portion whereupon the two EPAMs 111a and 111b are attached have a protruding portion 113a fit therein, which is provided on the inner circumferential surface of the operating unit side cap 113, as shown in Fig. 16, and as well as position-determining in the circumferential direction, the rotation is also restricted so that the cap 102 is not inadvertently rotated.

An electrode 114 is attached to the inner circumferential surface and the outer circumferential surface of each EPAM 111a and 111b (see Fig. 16), and are each connected to one end of the cables 103a and 103b. Each cable 104a and 103b are connected to the power source supplying unit 105 via the switches 104a and 104b which are each provided halfway on the cables.

This cap 102 is attached to the operating unit side cap 113, and for example when the switch 104b is turned ON, the cross-sectional view becomes as that in Fig. 16.

The EPAM 111b to which driving voltage is applied expands in the circumferential direction. In this case, expansion of the end portion on the bottom side shown in Fig. 16 of the EPAM 111b is restricted by the protruding portion 113a of the operating unit side cap 113, and therefore the end portion on the opposite side pressures to move the cap 102 in a counter clockwise direction, accordingly the cap 102 rotates in a counter-clockwise direction. In this case, the other EPAM 111 a is constricted in the circumferential direction by the pressure force in the event that the EPAM 111b expands.

When the switch 104a is turned ON, this becomes an action of replacing the EPAM 111b with the 111a, thus in this case the cap 102 rotates in the clockwise direction.

The insertion portion 6 can be easily twisted in the case of this modification example as well. According to the present modification example, the EPAMs 111a and 111b are provided only on the cap 102 portion, so that almost the same configuration as that in Fig. 13 can be realized with lower cost.

### (Fourth Embodiment)

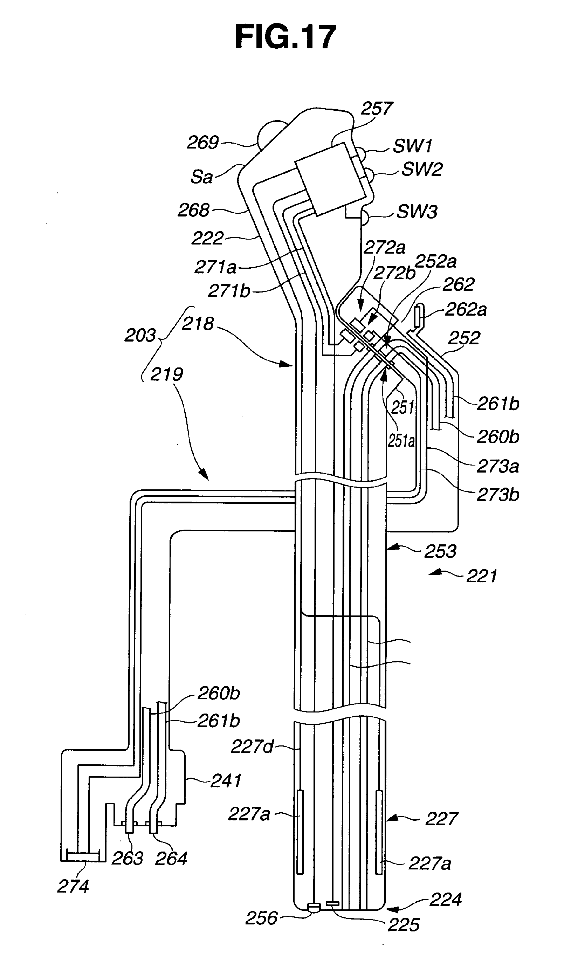

Next, a fourth embodiment of the present invention will be described with reference to Fig. 17 through Fig. 29.

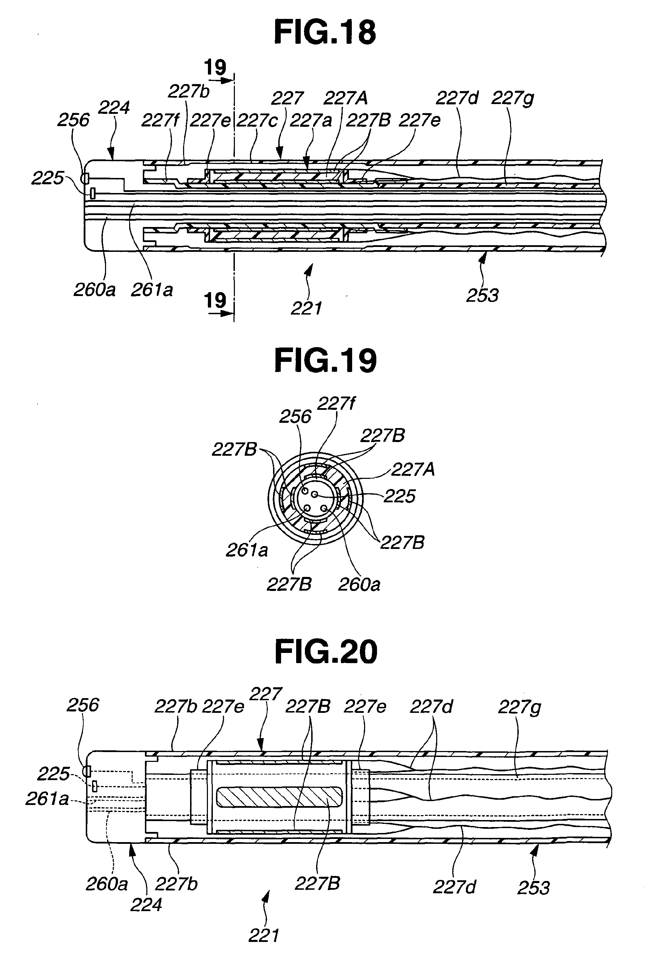

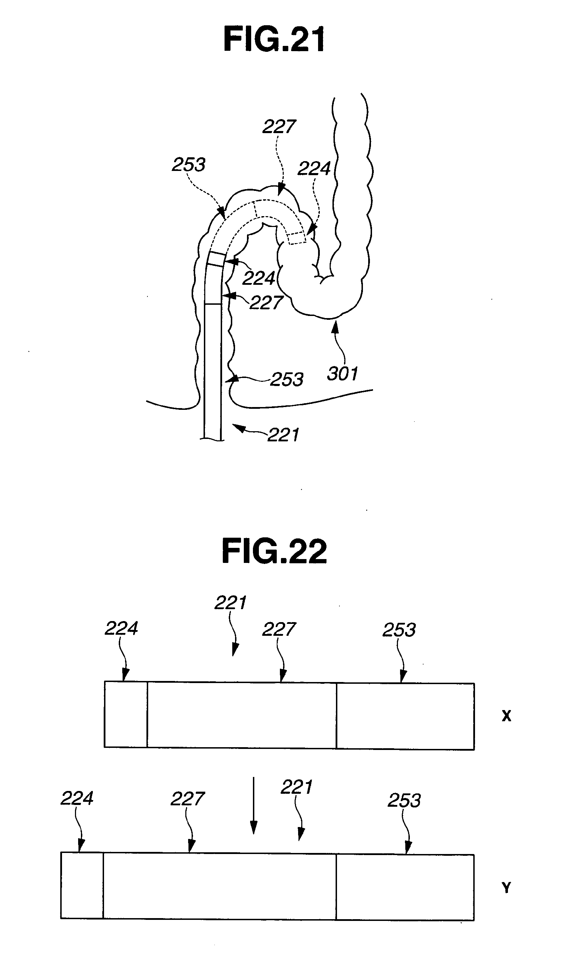

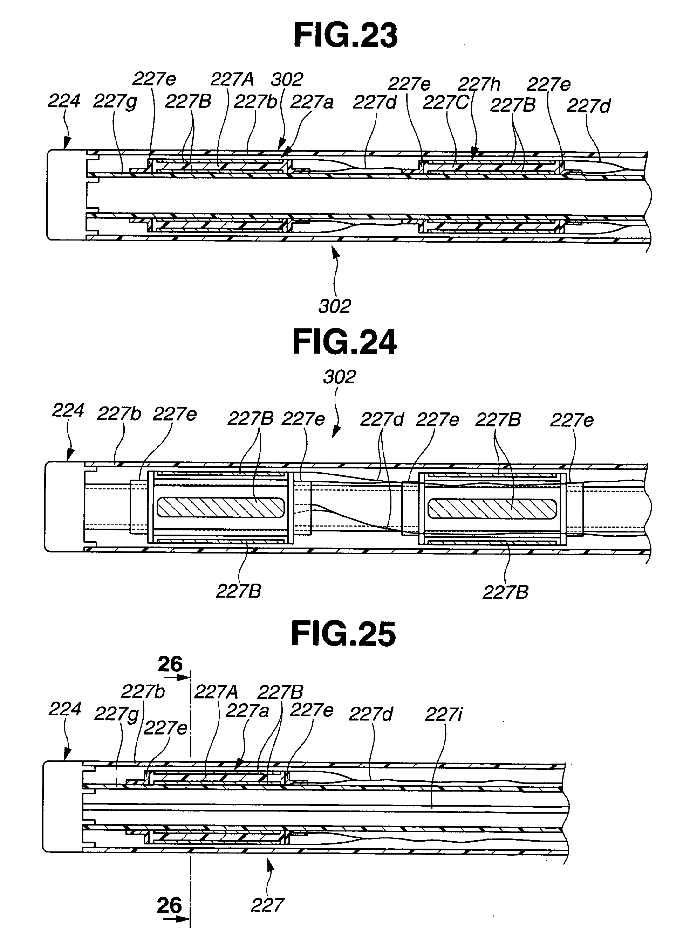

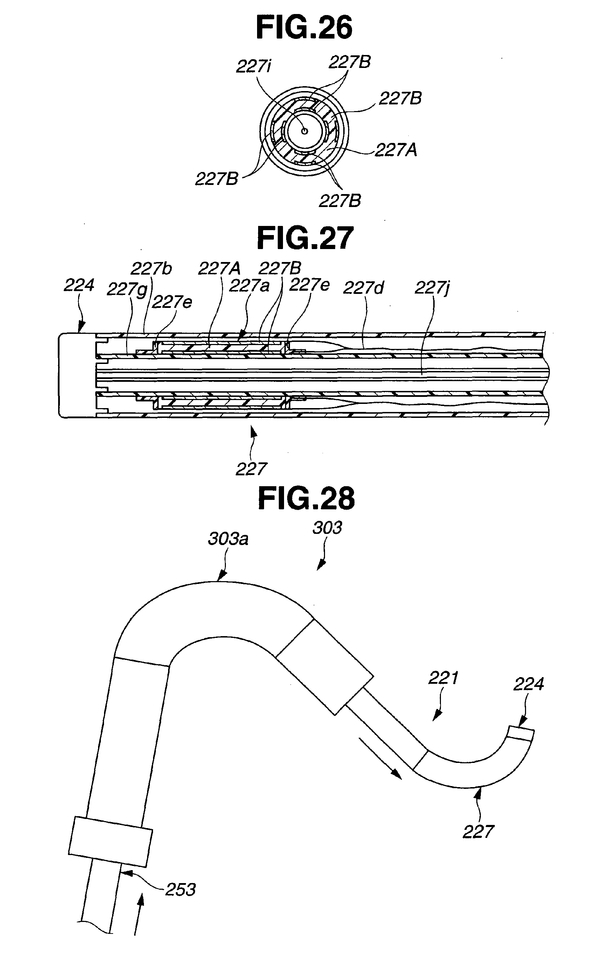

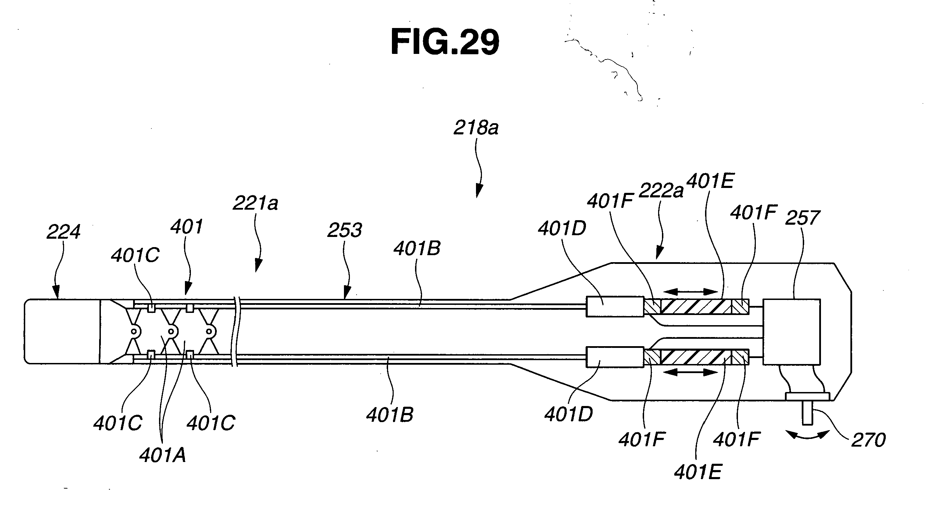

Fig. 17 is an overall configuration diagram of the endoscope according to the present embodiment. Fig. 18 is a cross-sectional view in the insertion axis direction of the endoscope of a bending portion of the endoscope relating to the present embodiment. Fig. 19 is a cross-sectional view along the 19-19 line in Fig. 18. Fig. 20 is a cross-sectional view in the insertion axis direction of the endoscope in the case of showing a cross-section of the external tube portion in Fig. 18. Fig. 21 is a diagram showing the state of the endoscope according to the present embodiment being inserted into a body cavity. Fig. 22 is a diagram showing the change in shape of a bending portion in the event of applying a generally similar voltage to all electrodes provided on the bending portion of the endoscope according to the present embodiment. Fig. 23 is a cross-sectional view in the insertion axis direction of the endoscope in the case of having two bending portions which bend in four directions provided, in the bending portion of the endoscope according to the present embodiment. Fig. 24 is a cross-sectional view in the insertion axis direction of the endoscope in the case of showing a cross-section of the external tube portion in Fig. 23. Fig. 25 is a cross-sectional view in the insertion axis direction of the endoscope in the case of having a core line provided in the generally central portion within an inner tube, in the bending portion of the endoscope according to the present embodiment. Fig. 26 is a cross-sectional diagram along the 26-26 line in Fig. 25, in the case of having a core line provided in the generally central portion within an inner tube, in the bending portion of the endoscope according to the present embodiment. Fig. 27 is a cross-sectional view in the insertion axis direction of the endoscope, in the case of having a pipe provided in the generally central portion within an inner tube, in the bending portion of the endoscope according to the present embodiment. Fig. 28 is a diagram illustrating a configuration in the case of applying the bending mechanism provided on the bending portion of the endoscope according to the present embodiment to a sliding tube. Fig. 29 is a schematic diagram of the configuration of the endoscope main unit as a modification example of the endoscope according to the present embodiment.

The endoscope 203 according to the present embodiment comprises, as shown in Fig. 17, an endoscope main unit 218 having a flexible insertion portion 221 and an operating unit 222 which is provided on the rear end of the insertion portion 221, and a tube unit 219. The tube unit 219 is of a disposable type, and is configured such that an integrated connector portion 252 provided on the base end is detachably connected to the connector portion 251 provided near the base end of the operating unit 222. Also, a scope connector 241 which is detachably connected to an unshown AWS (air / water / suction) unit is provided on the terminal end of the tube unit 219.

The insertion portion 221 comprises a rigid tip portion 224 provided on the tip of the insertion portion 221, a bendable bending portion 227 provided on the rear end of the tip portion 224, and a slender flexible tube portion 253 provided on a portion from the rear end of the bending portion 227 to the operating unit 222.

An LED 256, for example, is attached to the inner side of the illumination window provided on the tip portion 224 of the insertion portion 221 as illumination means. The illumination light emitted from the LED 256 is emitted toward the front via an illumination lens attached so as to be integrated with the LED 256, and illuminates the portion to be treated and so forth serving as a subject.

Note that the LED 256 may be configured with an LED generating white light, or R LED, G LED, and B LED for generating the wavelengths of light each for red (R), green (G), and blue (B) may be used. The light generating element forming the illumination means is not limited to the LED 256, but an LD (laser diode) or the like may also be used to form the light generating element. Further, instead of the LED256, illumination means which are configured with light guiding means such as a light guide fiber or the like provided so as to be inserted through the tube unit 219 and insertion portion 221, and light source means for irradiating illumination light on the light guiding means may also be used.

Also, the image-capturing means for image-capturing a subject is made up of an unshown objective lens which is attached to the observation window provided adjacent to the illumination window, and a CCD 225 which is disposed in an image-forming position of the objective lens and which has the function to vary the gain.

With the CCD 225 according to the present embodiment, the CCD device itself has the function for varying the gain, and can easily vary the gain of the CCD output signal up to several hundred times with this gain varying function. Therefore, even under the illumination light by the LED 256 a light image with little S/N decrease can be obtained.

One end of a signal line is connected to the LED 256 and the CCD 225, and the other end of the signal line which is inserted into the insertion portion 221 is provided within the operating unit 222 for example, and connected to the control circuit 257 which performs concentrated control processing.

An EPAM (Electroactive Polymer Artificial Muscle) actuator 227a serving as a bending mechanism made up of an EAP (Electroactive Polymer) 227A serving as a electroconductive expanding/contracting member having a center axis parallel to the insertion axis, and an electrode 227B, is provided on the inner side of the outer skin of the bending portion 227. Also, the EPAM actuator 227a is connected to a control circuit 257 via a control line 227d. Note that detailed configuration and so forth of the bending portion 227 including the EPAM actuator 227a will be described later.

Also, an air/water-sending tube 260a and a suction tube 261 a are inserted in the insertion portion 221, and the rear end portion thereof has an opening at the connector portion 251, and comprises a portion of the tube connector portion 251a. Also, the tube connector portion 251a is detachably connected to the tube connector 252a of the integrated connector unit 252 which is provided on the base end of the tube unit 219.

When the tube connector portion 251a and the tube connector 252a are connected, the air/water-sending tube 260a is connected to the air/water-sending tube 260b which is inserted in the tube unit 219. The suction tube 261 a is connected to the suction tube 261b which is inserted in the tube unit 219, and also divides within the tube connector 252a and opens to the outside, and links to a forceps opening 262 serving as a treatment tool insertion opening wherein a treatment tool such as a forceps can be inserted. This forceps opening 262 is closed off by a forceps plug 262a when not in use.

The rear ends of the air/water-sending tube 260b and the suction tube 261b are configured as an air/water sending cap 263 and a suction cap 264 with the scope connector 241.

Also, as shown in Fig. 17, a gripping portion 268 for the surgeon to grip is provided on the operating unit 222 of the endoscope main unit 218. Also, for example three scope switches SW1, SW2, and SW3 which perform remote control operations such as releasing and freezing are provided along the axis in the lengthwise direction of the operating unit 222 on the gripping portion 268 or in the periphery thereof, and each are connected to the control circuit 257.

Further, a track ball 269 with a water-resistant configuration is provided in a position whereby the surgeon can perform operations with the hand gripping the gripping portion 268, on a diagonal face portion Sa which is formed by a diagonal upper surface on the opposite side from where the scope switches SW1, SW2, and SW3 are provided on the operating unit 222. The track ball 269 is connected to the control circuit 257, and the surgeon can perform settings and so forth of bending operations or remote control operations by turning the track ball 269.

A power source line 271 a and signal line 271b which are connected to the control circuit 257 are electrically connected without contact to a power source line 273a and signal line 273b which are provided so as to be inserted through the tube unit 219, via non-contact transfer portions 272a and 272b which are formed on the connector portion 251 and the integrated connector portion 252. The power source line 273a and signal line 273b are connected to an electric connector 274 having a power source and signal contact point on the scope connector 241.

Next, the detailed configuration of the bending portion 227 provided on the endoscope main unit 218 will be described with reference to Fig. 18 through Fig. 20.

The bending portion 227 comprises an EPAM actuator 227a which is bendable in the four directions, up, down, left, and right, as to the insertion axis of the endoscope 203, an outer tube 227b made of a material such as a resin or the like, an attaching flange 227c provided for attaching the EPAM actuator 227a, and an expanding/contracting member 227f which is generally in a tube shape and which is provided further on the inner side than the EPAM actuator 227a. A portion near the EPAM actuator 227a which is also a portion of the outer tube 27b is thinly formed as a thin portion 227c, as compared to other portions of the outer tube 27b, such that the bending portion 227 easily expands/contracts. Also, the expanding/contracting member 227f expands/contracts well, and also is configured from a member such as a resin with high electrical insulation.

The EPAM actuator 227a is in a general tube shape having a predetermined length along the insertion axis direction of the endoscope 203, and is configured with an EAP 227A serving as an electroconductive member which can change the shape of the bending portion 227 by expanding/contracting in the insertion portion direction of the endoscope 203 by voltage being applied thereto, and four pairs of electrodes 227B, each having a thin plate shape, which are provided in positions facing one another and sandwiching the EAP 227A, and which is for applying voltage to the EAP 227A. Also, the four pairs of electrodes 227B are each provided in position corresponding to the up, down, left, right directions of the bending directions. Also, a control line 227d maintains a electrically insulated state as to each member provided on the inner tube 227g, and is connected to each of the four pairs of electrodes 227B. Also, as shown in Fig. 18, a signal line, air/water-sending tube 260a and so forth connected to the CCD 225 are provided on the inner portion of the inner tube 227g made up of a resin or the like with a high electrical insulation, which is provided so as to be connected to the expanding/contracting member 227f and to the rear end portion of the expanding/contracting member 227f. Also, Fig. 19 is a cross-sectional view along the 19-19 line in Fig. 18, and Fig. 20 is a cross-sectional view in the insertion axis direction of the endoscope in the case of showing a cross-section of the external tube portion in Fig. 18.

Next, the operations and so forth of the bending portion 227 when the insertion portion 221 of the endoscope main unit 218 of the present embodiment is inserted into a body cavity will be described.

When the insertion portion 221 of the endoscope main unit 218 is inserted into a body cavity 301 of a live body, there are situations wherein insertion of the endoscope main unit 218 gets caught and cannot be inserted smoothly, as shown in Fig. 21. In such cases, first the surgeon rotates the track ball 269 in the direction in which the surgeon desires to bend the bending portion 227 as to the insertion axis of the endoscope 203 (the right direction in the case shown in Fig. 21). When the surgeon rotates the track ball 269, a bending operation signal is output as to the control circuit 257, based on this rotation. The control circuit 257 outputs a bending control signal having a voltage value according to the bending amount, as to the electrodes 227B of the EPAM actuator 227a which corresponds to the bending direction of the bending operation signal instructions, based on the bending operation signal output from the track ball 269. Then the electrodes 227B apply voltage to the EAP 227A, based on the bending control signal. In the situation shown in Fig. 21, the insertion of the insertion portion 221 becomes smoother by bending the bending portion 227 in the right direction as to the insertion axis when viewed toward the paper. Therefore, by applying voltage to one pair of electrodes 227B provided on the left side of the insertion axis, of the four pairs of electrodes 227B provided in the bending portion 227, the left side face of the EAP 227A is expanded, and the bending portion 227 is bent in the right direction of the insertion axis. Thus, the bending portion 227 is bent in a shape shown with a dotted line in Fig. 21, and so the insertion portion 221 can be smoothly inserted into deep portions of the body cavity 301.

Note that if an operation can be performed for applying generally the same voltage to the EAP 227A from all of the electrodes provided on the bending portion 227, that is to say from the four pairs of electrodes 227B provided on the bending portion 227 of the insertion portion 221, as shown in Fig. 22, the bending portion 227 expands in the insertion axis direction from the state of X to the state of Y. Therefore, the position of the insertion portion 221 within the body cavity can be fixed, and from this position even further deeper into the body cavity can be observed.

Further, as shown in Fig. 23, the insertion portion 221 of the endoscope main unit 218 may also have a bending portion 302 with multiple bending mechanisms provided at locations wherein vertical and horizontal bending can be performed as to the insertion axis of the endoscope 203. Note that with the insertion portion 221 shown in Fig. 23 through Fig. 27, the configuration of the tip portion 224 and within the inner tube 227g is the same as the configuration in Fig. 18 through Fig. 20, and therefore detailed description will not be made hereafter, nor will diagrams be shown. Also, with the insertion portion 221 shown in Fig. 23 through Fig. 27, the outer tube 227b is assumed to be formed with generally the same thickness for ease of description, and further, the expanding/contracting member 227f is assumed to be integrated with the inner tube 227g.

The bending portion 302 has a configuration of the above-described bending portion 227, and also has an EPAM actuator 227h serving as a bendable mechanism wherein bending in the four directions of up, down, right, left as to the insertion axis of the endoscope 203 can be performed on the inner side of the outer skin in locations nearer to a flexible tube 253 than the EPAM actuator 227a. The EPAM actuator 227h is configured with an EAP 227C serving as an electroconductive member and four pairs for electrodes 227B for applying voltage to the EAP 227C which are provided in a position facing one another and sandwiching the EAP 227C. Note that the control line 227d which is connected to each of the EPAM actuator 227a and the electrodes 227B of the EPAM actuator 227h is provided on the outer circumferential surface of the inner tube 227g so as to crawl thereon, for example, so that an electrically insulated state can be maintained for each member provided on the inner portion of the inner tube 227g. Also, Fig. 24 is a cross-sectional view in the insertion axis direction of the endoscope 203 in the case of showing a cross-section of the external tube portion in Fig. 23.

Also, as shown in Fig. 25, a core line 227i may be provided in the generally central portion of the inner portion of the inner tube 227g, in the bending portion 227 of the endoscope main unit 218. The core line 227i is made up of a highly elastic material such an Ni-Ti alloy or the like, and by providing this in the generally central portion of the inner portion of the inner tube 227g, distortion can be lessened when the surgeon inserts the bending portion 227 into the body cavity, and thus insertion of the insertion portion 221 into the body cavity can be performed smoothly. Fig. 26 is a cross-sectional diagram along the 26-26 line in Fig. 25, in the case of having a core line 227i provided in the generally central portion within the inner tube 227g of the bending portion 227. Note that as shown in Fig. 27, instead of providing a core line 227i, a pipe 227j made of a material such as Teflon (registered trademark) may be provided in the bending portion 227. Also, the pipe 227j may be duplicated as an air/water-sending tube 260a or a suction tube 261a.

Also, as shown in Fig. 28, a sliding tube 303 for performing smooth insertion of the insertion portion 221 into the body cavity may have a sliding tube bending mechanism 303a of a configuration similar to the EPAM actuator 227a.

With the endoscope 203 according to the present embodiment, the EPAM actuator 227a provided on the bending portion 227 is formed with the EAP 227A and four pairs of electrodes 227B. Thus, compared to a conventional bending mechanism, the endoscope itself can be made to be more lightweight, since there is no need to provide bending pieces, bending wires, and so forth. As a result, the surgeon can perform a treatment using an endoscope for a longer continuous period of time.

Also, the endoscope main unit 218a, as a modification example of the present embodiment, has a flexible insertion portion 221 a and an operating unit 222a provided on the rear end of the insertion portion 221a, as shown in Fig. 29.

The insertion portion 221a comprises a tip portion 224 which is provided on the tip of the insertion portion 221a and which has a similar configuration as the above-described configuration, a bendable bending portion 401 provided on the rear end of the tip portion 224, and a slender flexible tube portion 253 which is provided to the portion from the rear end of the bending portion 401 to the operating unit 222a. Also, a signals line, tube, and so forth which have similar configurations as the inner portion of the above-described inner tube 227g are provided inside the insertion portion 221a by being inserted through the insertion portion 221a.

The bending portion 401 configuring a portion of the bending mechanism is provided so as to be inserted through one or multiple bending pieces 401A, the insertion portion 221a, and the operating unit 222a, and is configured with two bending wires 401B which are connected to the two ends of circumferential portions of the bending pieces 401A, and a wire receiver 401C for connecting the bending pieces 401A and the bending wires 401B, in order to rotate the bending pieces 401A.

The operating unit 222a is provided so as to be connected to one end of each of the two bending wires 401B as a portion of the bending mechanism, wherein an EAP401E serving as an electroconductive member and which can tighten or relax the bending wires 401B, and an electrode 401F provided to apply voltage to the EAP 401E, are provided therein. Also, the electrode 401F is connected to the control circuit 257 within the operating unit 222a. Also, the operating unit 222a includes a linking member 401D provided for connecting the bending wires 401B and the EAP 401E. Note that if the EAP 401E is provided in a position to be connected to one end of the bending wires 401B, the EAP401E does not need to be provided within the operating unit 222a, and for example may be provided near the base end of the flexible tube portion 253.

Further, the operating unit 222a has a joystick 270 for operating the bending mechanism comprising the bending portion 401, the EAP401E, and the electrode 401F provided on the exterior surface thereof, and is connected to the control circuit 257 within the operating unit 222a.

Next, the operations and so forth of the bending mechanism comprising the bending portion 401, the EAP401E, and the electrode 401 F, in the event of inserting the insertion portion 221 a of the endoscope main unit 218a of the present modification example into a body cavity will be described.

In the event of inserting the endoscope main unit 218a into a body cavity, for example as shown in Fig. 21, in a case wherein the endoscope main unit 218a is caught in a location preventing smooth insertion, first, the surgeon operates the joystick 270 in the desired direction of the bending portion 401 to be bend as to the insertion axis of the endoscope 203 (in the right direction in the situation shown in Fig. 21). When the surgeon operates the joystick 270, a bending operation signal is output to the control circuit 257, based on this operation. The control circuit 257 outputs a bending control signal having a voltage value according to the bending amount to the electrode 401F, based on the bending operating signal output from the joystick 270. Then the electrode 401F applies voltage to the EAP 401 E, based on the bending control signal. For example, in a situation as shown in Fig. 21, by bending the bending portion 401 toward the right direction of the insertion axis, the insertion portion 221 a can be inserted smoothly. Therefore, the control circuit 257 applies voltage to the EAP401E provided on the left side of the insertion axis of the endoscope 203, via the electrode 401F. The EAP 401E expands when voltage is applied, and the bending wires 401B provided on the left side of the insertion axis of the endoscope 203 are relaxed via a linking member 401D. The bending wires 401B which are now in a relaxed state bend the bending portion 401 in a shape such as that shown with dotted lines in Fig. 21, by rotating the bending pieces 401A. As a result, the insertion portion 221a can be inserted smoothly into a deep portion within the body cavity.

The endoscope main unit 218a serving as a modification example of an embodiment of the present invention has an EAP401E and electrode 401F, serving as a portion of the bending mechanism, provided on the operating unit 222a. Thus, compared to a conventional bending mechanism, the endoscope itself can be made more lightweight since there is no need to provide a motor and so forth for pulling the bending wires. As a result, the surgeon can perform treatment using an endoscope for a longer consecutive time period, with less physical burden than with a conventional situation.

Note that the present invention is not limited to the above-described embodiments, and it goes without saying that various modifications and applications can be made without departing from the spirit and scope of the present invention.

The present application claims a priority from Japanese Patent No. 2004-260133 filed in Japan on September 7, 2004, and Japanese Patent No. 2004-260131 filed in Japan on September 7, 2004. The contents of the disclosures are cited in the specification of the present application, claims, and the drawings alike.

## Claims

1. An endoscope comprising:
an insertion portion for being inserted into a subject body cavity;
an electroconductive expanding/contracting member which can expand and contract the insertion portion in the lengthwise direction, and which expands and contracts according to a voltage application state; and
electrodes for applying voltage supplied from a power source to the electroconductive expanding/contracting member.

2. An endoscope having a variable rigidity device for changing the rigidity of an insertion portion, such variable rigidity device comprising:
a variable rigidity member disposed along the lengthwise direction of the insertion portion and the rigidity thereof is changed by being compressed;
compression applying means for applying compression force to the variable rigidity member according to an electrical signal to be applied; and
instruction operating means for performing instruction operations as to the compression applying means.

3. The endoscope according to Claim 2, wherein a plurality of the variable rigidity members are disposed along the lengthwise direction of the insertion portion.

4. The endoscope according to Claim 3, wherein the instruction operating means generate a instruction signal for modifying the rigidity of the variable rigidity member in an arbitrary position, a plurality of the variable rigidity members being disposed along the lengthwise direction of the insertion portion.

5. The endoscope according to Claim 2, wherein the compression applying means are configured using artificial muscle with adjustable compression force which is to be applied to the variable rigidity member, according to the value of the applied electrical signal.

6. The endoscope according to Claim 2, wherein the instruction operating means are provided in the periphery of a gripping unit at the rear end of the operating unit or the insertion portion.

7. The endoscope according to Claim 2, wherein the compression applying means have the function of the variable rigidity member.

8. The endoscope according to Claim 3, wherein the compression applying means are formed adjacent to and divided among each of a plurality of variable rigidity members which are disposed along the lengthwise direction of the insertion portion so that the compression force can be applied to each variable rigidity member.

9. The endoscope according to Claim 8, wherein the compression applying means are formed adjacent to and divided among each variable rigidity member, in the lengthwise direction thereof.

10. The endoscope according to Claim 8, wherein the compression applying means are formed into a plurality of pieces in the inner circumferential direction of an exterior tube of the insertion portion, and each of the divided compression applying means also has the function of each variable rigidity member.

11. The endoscope according to Claim 2, wherein the compression applying means are connected to electrical signal generating means for generating the electrical signal.

12. The endoscope according to Claim 2, wherein the instruction operating means are connected to control means for performing control of electrical signal applying as to the compression applying means, by an instruction signal from the instruction operating means.

13. The endoscope according to Claim 12, wherein the control means perform processing for generating information displaying the rigidity which is set by the instruction signal by the instruction operating means.

14. The endoscope according to Claim 2, wherein a protruding mechanism in which protruding portion can be retractable is disposed on at least one position on the exterior surface of the insertion portion.

15. An endoscope having a slender insertion portion, comprising:
a protruding mechanism as a protruding portion retractable by an electrical signal, in at least one position on the exterior surface of the insertion portion; and
instruction operating means for performing instruction operations of the protruding/retracting of the protruding portion.

16. The endoscope according to Claim 15, wherein the protruding mechanism has retractable protruding portions in a plurality of positions in the lengthwise direction of the insertion portion.

17. The endoscope according to Claim 15, wherein the protruding portion is configured with artificial muscle.

18. An endoscope having a slender insertion portion, comprising:
artificial muscle to be a pair for generating twisting force to twist the insertion portion with the electrical signal applied; and
selection means for selectively performing electrical signal applying operations for each of the pair of artificial muscles.

19. The endoscope according to Claim 18, wherein the pair of artificial muscles is formed with artificial muscles in a band shape wrapped around the inner circumferential surface of the outer skin of the insertion portion in a spiral, wrapped in the right direction and wrapped in the left direction.

20. The endoscope according to Claim 18, wherein the pair of artificial muscles is formed being disposed in the circumferential direction of the cap at the rear end of the insertion portion.

21. An endoscope having an insertion portion with a bending portion, comprising:
an electroconductive expanding/contracting member of a predetermined length along the insertion axis direction of the endoscope, for expanding/compressing in the insertion axis direction of the endoscope by the voltage application state, to change the shape of the bending portion; and
at least one pair of electrodes provided in positions facing each other with the electroconductive expanding/contracting member in between the two, for the purpose of applying voltage to the electroconductive expanding/contracting member.

22. The endoscope according to Claim 21, wherein the electroconductive expanding/contracting member is formed with an electroconductive polymer member.

23. An endoscope having an insertion portion with a bending portion and an operating unit, the endoscope comprising:
one or a plurality of bending pieces provided on the bending portion;
a bending wire which is connected to at least one end of the one or plurality of bending pieces, which is provided so as to be inserted in the insertion portion and the operating unit, in order to rotate the one or plurality of bending pieces provided on the bending portion;
an electroconductive expanding/contracting member which is provided so as to be connected to one end of the bending wire, and which expands/contracts based on the voltage application state to expand or contract the bending wire; and
an electrode provided on the electroconductive expanding/contracting member for the purpose of applying the voltage as to the electroconductive expanding/contracting member.
